# EUROPEAN PATENT SPECIFICATION

(11) **EP 0 478 696 B1**
(45) Date of publication and mention of the grant of the patent: **12.04.1995**
(21) Application number: 90911172.6
(22) Date of filing: 22.06.1990
(51) Int. Cl.: C12P 21/06, C07K 14/00

(54) **EFFICIENT METHOD FOR IDENTIFIABLE EXPRESSION OF NON-SELECTABLE GENES**
EFFIZIENTES VERFAHREN ZUR NACHWEISBAREN EXPRESSION NICHTSELEKTIERBARER GENE
PROCEDE EFFICACE POUR UNE EXPRESSION IDENTIFIABLE DE GENES NON SELECTIONNABLES

(30) Priority: 23.06.1989 US 370619
(43) Date of publication of application: 08.04.1992
(73) Proprietor: THE UNITED STATES OF AMERICA, represented by THE SECRETARY, UNITED STATES DEPARTMENT OF COMMERCE, Washington, DC 20231 (US)
(72) Inventor: KANE, Susan, Elizabeth/City of Hope Medical, Duarte, CA 91010 (US); PASTAN, Ira, Harry, Potomac, MD 20854 (US); GOTTESMAN, Michael, Marc, Bethesda, MD 20817 (US)
(74) Representative: Jump, Timothy John Simon
(86) International application number: US9003524
(87) International publication number: WO9100361

(56) References cited:
- EP-A- 162 699
- US-A- 4 816 565
- JOURNAL OF BIOLOGICAL CHEMISTRY, vol. 264, no. 13, 05 May 1989, American Society of Molecular Biology Inc., US; U.A. GERMANN et al., pp. 7418-7424#
- MOLECULAR & CELLULAR BIOLOGY, vol. 8, no. 8, August 1988, American Society of Microbiology, Washington, DC (US); S.E. KANE et al., pp. 3316-3321#
- PROCEEDINGS OF THE NATL. ACADEMY OF SCIENCES USA, vol. 84, May 1987, US; UEDA et al., pp. 3004-3008#
- JOURNAL OF CELLULAR BIOCHEMISTRY ABSTRACTS, UCLA Symposia on Molecular & Cellular Biology, 15th ann. Meetings, 20 January to 15 February 1986; CLARK et al., abstract#
- NATURE, vol. 323, October 1986; GROS et al., pp. 728-731#
- GENE, vol. 84, December 1989; KANE et al., pp. 439-446#

## Description

The present application illustrates the construction of a cloned multidrug resistance gene and various uses thereof, and provides means for linking in the same vector, a non-selectable gene desired to be expressed, with the dominant selectable multidrug resistance gene MDR1, so that the selection for MDR1 reliably and simultaneously allows the amplification and over-expression of the non-selectable gene.

These and other objects, features and many of the attendant advantages of the invention will be better understood upon a reading of the following detailed description when considered in connection with the accompanying drawings wherein:

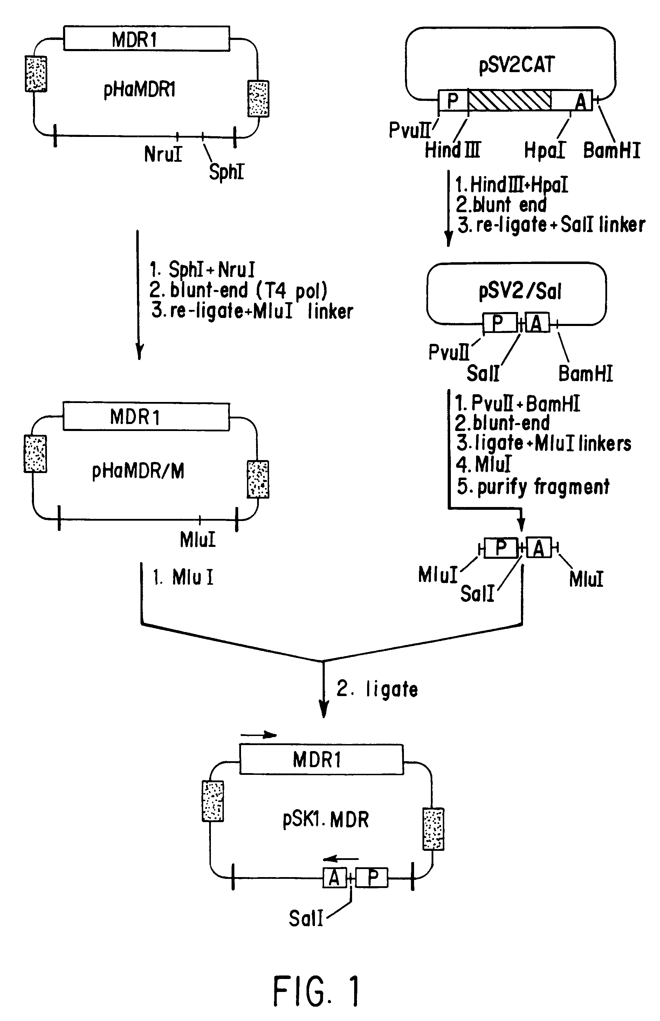

Figure 1 shows a schematic construction of the pSK1.MDR expression vector. pHaMDR1 was modified by removing pBR322 sequences between the Nru1 and Sph1 sites and replacing them with a MluI linker (pHaMDR/M). pSV2CAT was modified by removing the CAT coding sequences and replacing them with a SalI linker (pSV2/Sal). The unit containing the SV40 promoter and polyadenylation signal flanking the Sal I site was removed from pSV2/Sal. MluI linkers were added to the ends of that unit, and it was cloned into the MluI site of pHaMDR/M. The final plasmid was designated pSK1.MDR. Grey boxes represent retroviral long terminal repeats and the MDR1 open box is a human MDR1 cDNA fragment coding for the multidrug transporter. The hatched box in pSV2CAT is the CAT coding region. The box designated P is the SV40 promoter + enhancer element. The box designated A contains the polyadenylation signal from SV40. Arrows indicate direction of transcription.

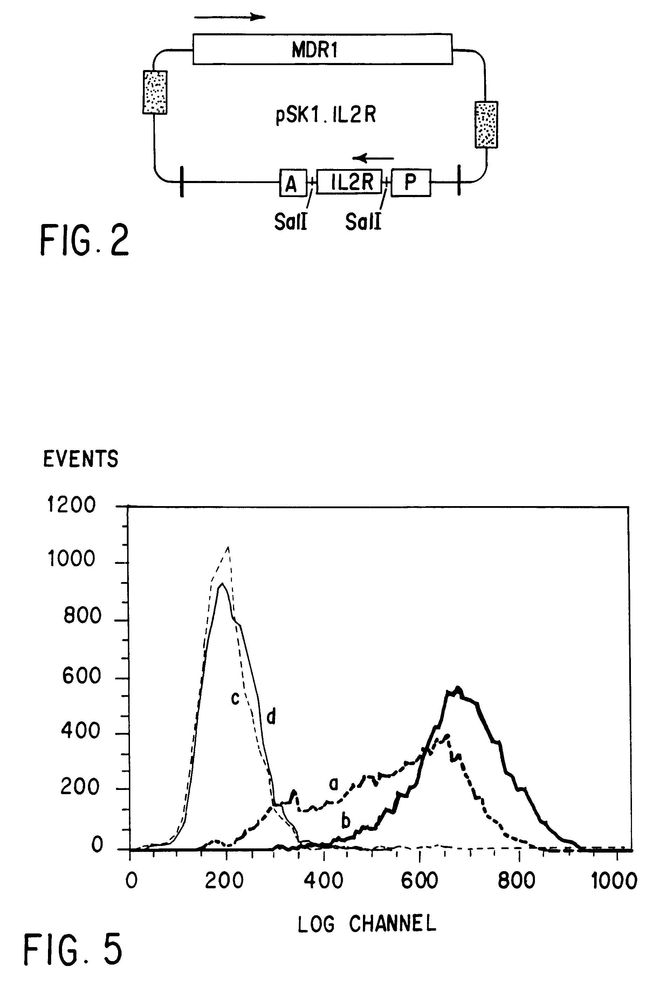

Figure 2 is a schematic representation of pSK1.IL2R. SalI linkers were added to the ends of a DNA fragment containing the entire coding region of the p55 subunit of the interleukin-2 receptor (IL2R). The fragment was then cloned into the SalI site of pSK1.MDR. A clone containing the IL2R insert in the positive ("sense") orientation relative to the SV40 promoter (the direction of transcription is indicated by arrows) was designated pSK1.IL2R. A clone with the insert in the opposite orientation was called pSK1.R2LI.

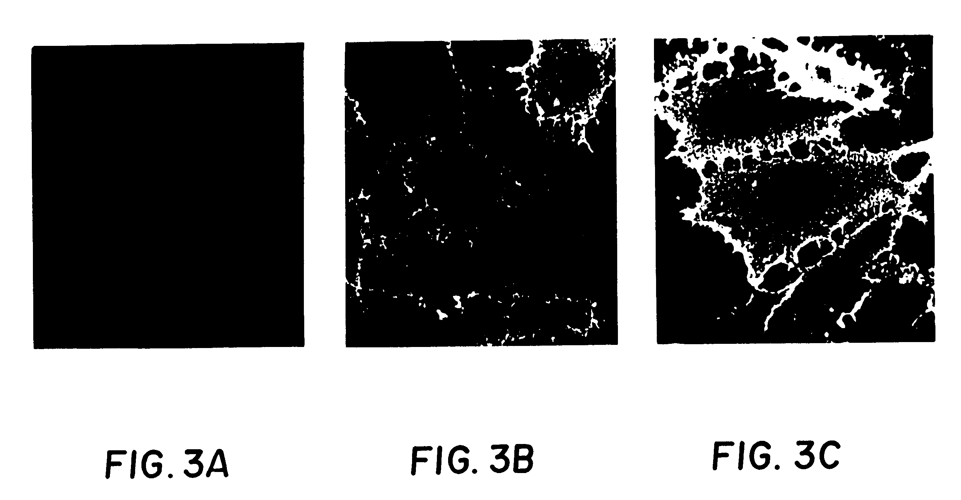

Figures 3A, 3B and 3C demonstrate the results of immunofluorescence. Cells transfected with pSK1.R2LI (Fig. 3A) or with pSK1.IL2R (Figs. 3B and 3C) and growing in 80 ng/ml colchicine (Figs. 3A and 3B) or in 640 ng/ml colchicine (Fig. 3C) were stained for cell-surface expression of the IL2R. Shown are representative fields from each sample, which were photographed with a Zeiss photomicroscope, 400X magnification.

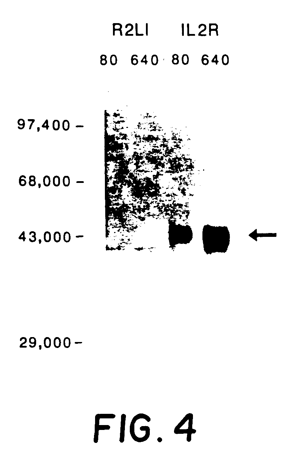

Figure 4 shows the results of immunoprecipitation. Transfected cells were labeled and immunoprecipitations performed. The lanes marked R2LI represent lysates from control cells transfected with the pSK1.R2LI (opposite orientation) plasmid and growing at 80 or 640 ng/ml colchicine. The lanes marked IL2R are lysates from pSK1.IL2R cells, at 80 or 640 ng/ml colchicine. The numbers on the left indicate the position of molecular weight markers. The arrow points to the p55 protein specifically immunoprecipitated by anti-Tac monoclonal antibody.

Figure 5 presents the results obtained from flow cytometry. Cells were prepared and analyzed using a FACScan with an argon laser (488 nm). The x-axis represents fluorescence intensity and the y-axis the number of cells. The thick gray line = pSK1.IL2R, 80 ng/ml colchicine; the thick black line = pSK1.IL2R, 640 ng/ml colchicine; the thin gray line = pSK1.R2LI, 80 ng/ml colchicine; the thin black line = pSK1.R2LI, 640 ng/ml colchicine.

Various objects and advantages of the present invention are achieved by the construction of a unique plasmid expression vector designated herein as pSK1.MDR which allows the expression of non-selectable genes, particularly in any suitable eukaryotic cells, some examples of such suitable eukaryotic cells being human HeLa cells, LoVo cells, CHO cells, HT-29 cells, SK-Hep cells, A431 cells and the like.

Accordingly, in a first aspect of the present invention, there is provided a recombinant plasmid expression vector comprising a human MDR1 gene at a first site and a second separate site for the insertion of a non-selectable gene, wherein when said non-selectable gene is inserted in said second separate site in the expression vector, and a cell is transfected with the plasmid vector containing said non-selectable gene, said non-selectable gene is simultaneously amplified and overexpressed along with the MDR1 gene.

In a second aspect of the present invention there is provided a cell transfected with a plasmid vector according to the invention in its first aspect or in any embodiment thereof.

In a third aspect of the present invention, there is provided a method for identifiable expression of a non-selectable gene, comprising inserting the cDNA of a non-selectable gene desired to be expressed into a plasmid expression vector according to claim 1, transfecting said expression vector into cells and identifying the expression of said non-selectable gene in said cells by the dominant selectable MDR phenotype with which said non-selectable gene is simultaneously overexpressed.

Preferred embodiments of the present invention in any of its aspects are as defined in the sub-claims.

For Contracting State ES, the invention relates to the methods and preferred embodiments thereof set out in the separate set of claims for that state.

The materials, methods and examples are illustrative only and not limiting.

The terms "non-selectable" or "non-selected" gene as used herein means a gene which is desired to be expressed, but which in itself does not have a selectable phenotype.

### Cells

NIH 3T3 cells were obtained from Dr. C. Scher, University of Pennsylvania School of Medicine and maintained in Dulbecco modified Eagle medium containing 10% calf serum (Colorado Serum Co.), 5 mM glutamine, 50 U of penicillin per ml, and 50 ug of streptomycn per ml. Colchicine was obtained from Sigma Chemical Co. and diluted from dimethyl sulfoxide stock solutions of 10 mg/ml to appropriate concentrations in complete medium.

### Plasmid constructions

The pHaMDR1 vector, described in the parent application, was first digested with SphI and NruI to remove sequences within the pBR322 portion of the plasmid (bp 562-972 on pBR322, including the SalI site at bp 651). NruI leaves a blunt end and the SphI end was made blunt with T4 DNA polymerase (Bethesda Research Laboratories). These ends were religated in the presence of MluI linkers, creating an intermediate plasmid with the indicated pBR322 sequences replaced with a unique MluI restriction site (pHaMDR/M). In a separate reaction, the pSV2CAT plasmid (Gorman et al, 1982, Mol. Cell Biol. 2, 1044-1051) was digested with HindIII and HpaI to remove the portion of the plasmid representing the chloramphenicol acetyltransferase gene. HpaI leaves a blunt end and the HindIII end was made blunt with the Klenow fragment of DNA polymerase I (Bethesda Research Laboratories). These ends were religated in the presence of SalI linkers, creating another intermediate plasmid (pSV2Sal) containing the desired transcription unit (the SV40 early promoter and the SV40 polyadenylation signal flanking a SalI site). pSV2Sal was digested with PvuII and BamHI to isolate the DNA fragment containing the transcription unit. PvuII leaves a blunt end and the BamHI end was made blunt with the Klenow fragment. MluI linkers were ligated onto the ends of this transcription unit and the purified fragment was in turn ligated into pHaMDR/M which had previously been digested with MluI. The resulting plasmid, pSK1.MDR, contains a unique SalI site for purposes of cloning foreign cDNAs within the SV40 transcription unit. This construction is illustrated in Fig. 1. pSK1.IL2R and pSK1.R2LI were constructed by adding SalI linkers to the ends of a 850 bp HindIII-XbaI fragment of IL2R cDNA (Leonard et al. 1985, J. Biol. Chem. 260, 1872-1880) which had been blunt-ended with Klenow, and then ligating the fragment into the SalI site of pSK1.MDR. pSK21.IL2R, illustrated in Fig. 2, contains the cDNA in the positive transcriptional orientation, pSK1.R2LI has the fragment in the negative orientation.

A deposit of the recombinant pSK1.MDR has been made at the ATCC, Rockville, Maryland on June 14, 1989, under the accession number ATCC 68011. The deposit shall be viably maintained, replacing if it becomes non-viable during the life of the patent, for a period of 30 years from the date of the deposit, or for 5 years from the last date of request for a sample of the deposit, whichever is longer, and upon issuance of the patent made available to the public without restriction in accordance with the provisions of the law. The Commissioner of Patents and Trademarks, upon request, shall have access to the deposit.

### Cell transfection and colchicine selection

pSK1.1L2R or pSK1.R2LI DNA was transfected into NIH 3T3 cells by the calcium phosphate coprecipitation method and cells were selected in the presence of colchicine as described by Kane et al (1988) Mol. Cell. Biol. 8, 3316-3321. Resulting colonies were pooled into 80 ng/ml colchicine and the concentration of colchicine was increased stepwise up to 1 »g/ml to select for amplified expression of the transferred MDR cDNA.

### Immunoprecipitations

Cells were labeled with 100 uCi of [³⁵S]methionine (Amershal Corp.) per ml in Dulbecco-Vogt medium lacking methionine (National Institutes of Health Media Unit) and supplemented with 5% calf serum, 5 mM glutamine, 50U of penicillin per ml, and 50 g of streptomycin per ml. Labeling was for 15 hrs and cells were lysed in RIPA buffer [20 mM Tris-HCl, pH 7.2, 0.15 M NaCl, 1% Triton X-100, 1% deoxycholate, 0.1% sodium dodecyl sulfate (SDS), 1% Aprotinin].

(Triton ® is a registered Trade Mark.). Immunoprecipitations were performed essentially as described by Kane et al, supra, Gal et al (1985) J. Cell Biol. 100, 535-544, using 5 x 10⁶ trichloroacetic acid-precipitable counts of cell lysate and anti-Tac, a mouse monoclonal antibody specific for the 55 kDa IL-2 receptor (Uchiyama et al, 1981, J. Immunol. 126, 1393-1397; Leonard et al, 1983, Proc. Natl. Acad, Sci. USA 80, 6957-6961). Secondary incubations were performed for 2 hrs at room temperature (about 22-24°C) with Protein A-Sepharose [20% in phosphate buffered saline (PBS) lacking calcium and magnesium], followed by washes as described by Gal et al, supra. Immunoprecipitates were run on a SDS-10% polyacrylamide gel as described by Laemmli (Laemmli, U.K., 1979, Nature (London) 227, 680-685). The gel was treated with 2,5-diphenyloxazole in dimethyl sulfoxide, and fluorography was performed at -70°C (Bonner et al, 1974, Eur. J. Biochem. 46, 83-88).

### Immunofluorescence

Populations of cells growing in 80 ng/ml colchicine or 640 ng/ml colchicine were seeded into 35 mm tissue culture dishes. Cells were stained live, first with the anti-Tac monoclonal antibody, followed by goat anti-mouse antibody conjugated to rhodamine (Jackson Immuno Research). Antibody incubations were for 30 min. at 4°C. The anti-Tac antibody concentration was 10 »g/ml and the anti-mouse antibody was 50 »g/ml. Both were in PBS with calcium and magnesium plus 2 mg/ml bovine serum albumin (BSA). After the final antibody incubation, cells were washed and then fixed for 10 min. at room temperature with 3.7% formaldehyde.

### Flow Cytometry

Flow cytometric analysis of the IL2R cell-surface marker was performed as follows. Cells were stained indirectly by incubating with the anti-Tac antibody followed by goat anti-mouse antibody conjugated with fluorescein (Jackson ImmunoResearch). Incubations were for 30 min. at room temperature, in PBS without calcium or magnesium plus 1% BSA. After the secondary antibody incubation, cells were fixed for 10 min. in 3.7% formaldehyde, washed, and resuspended at a final concentration of approximately 1 x 10⁶ cells per ml of PBS. Flow cytometry was performed using a FACScan (Becton-Dickinson) with an argon laser (488 nm). Surface receptor quantitation was done using a method described by LeBouteiller et al (1983) J. Immunol. Methods 61, 301-305, Monograph: Fluorescent Microbead Standards (1988) Flow Cytometry Standards Corporation, with standards purchased from Flow Cytometry Standards Corporation. Data analysis was done with FACScan Research software (Becton-Dickinson). Percent positive cells was estimated by determining the fraction of cells falling into a window of fluorescence intensity chosen to exclude 99% of the negative control cells.

### Construction of pSK1.MDR Expression Vector

The pHaMDR1 expression vector was modified by inserting a transcription unit consisting of the SV40 early region promoter and enhancer and the SV40 polyadenylation signal, flanking a unique SalI restriction site. The scheme for constructing this new plasmid, pSK1.MDR, is illustrated in Figure 1, and the details of the construction have been described herein above. An intermediate form of pHaMDR1 was prepared by removing sequences within the pBR322 portion of the plasmid (pb 562-972 on the pBR322 map, including the SalI site at position 651) and replacing them with a unique MluI site (pHaMDR/M). A DNA fragment containing an SV40 transcription unit, engineered with a SalI site between the SV40 promoter and the polyadenylation sequences, was ligated into the MluI site of pHaMDR/M. The resulting plasmid, pSK1.MDR, has the SV40 transcription unit inserted into the pBR322 region of the parent plasmid. The SalI site is unique and can be used as a cloning site for inserting foreign cDNAs. The direction of transcription for the MDR1 gene and for the SV40 transcription unit are indicated by arrows in Figures 1 and 2.

### Expression of a Foreign cDNA in pSK1.MDR

To test pSK1.MDR in transfection experiments, a cDNA for the interleukin-2 receptor (IL2R) was cloned into the SalI site of the SV40 transcription unit. The plasmid containing the IL2R (pSK1.IL2R) was transfected into NIH 3T3 cells by the calcium phosphate coprecipitation method (Shen et al, 1986, J. Biol Chem. 261, 7762-7770). As a control, the same cDNA was inserted into pSK1.MDR in the opposite transcriptional orientation (pSK1.R2LI) and transfected in parallel. Cells were selected for the ability to grow in 60 ng/ml of colchicine.

The initial analysis was performed on transfected cells which had been selected in 60 ng/ml of colchicine for two weeks and then pooled into the same selective medium for an additional 9 days. These populations were analyzed by immunofluorescence for the presence of IL2R surface antigen. An estimated 75-80% of the cells which were transfected with the pSK1.IL2R construction expressed significant amounts of IL2R on the cell surface (data not shown). The control cells transfected with pSK1.R2LI and selected in 60 ng/ml colchicine were completely negative for the IL2R antigen.

### Amplification of MDR1 and IL2R

Mixed populations of resistant cells (either pSK1.IL2R or pSK1.R2LI) were pooled into medium with 80 ng/ml of colchicine and then grown in increasingly higher concentrations of colchicine, up to 640 ng/ml, to amplify the expression of the transfected genes (Kane et al, supra). During this amplification process, the original population of cells growing in 80 ng/ml colchicine were maintained in culture at that same level of drug. By the time the final sets of analyses were performed, populations at 80 ng/ml colchicine had been in culture for 3-3.5 months, while the cells at 640 ng/ml had been growing at that drug concentration for 1-1.5 months. These groups of cells were then analyzed by three different methods to determine the level of IL2R expression in each group: immunofluorescence, immunoprecipitation and FACS analysis.

The results of the immunofluorescent staining are shown in Figure 3. Most of the cells in both the 80 ng/ml and the 640 ng/ml populations of pSK1.IL2R transfectants expressed IL2R on the cell surface. However, there were more cells in the low drug than in the high drug that appeared to express little or no cell surface IL2R. In addition, there was a higher percentage of cells expressing large amounts of IL2R in the 640 ng/ml culture than in the 80 ng/ml culture. There were, however, a significant number of cells in the 80 ng/ml population which stained very brightly for the cell surface IL2R antigen. Thus, even after 3 months in culture, under relatively mild selective conditions, some cells expressed a very large amount of non-selected gene (IL2R). Again, the control pSK1.R2LI cells were completely negative for IL2R staining.

To quantify better the expression of IL2R by each of these cell populations, immunoprecipitations were performed. Populations of cells were labeled metabolically with ³⁵S-methionine and IL2R was precipitated from whole cell extracts using the anti-Tac monoclonal antibody. As shown in Figure 4, cells growing in 640 ng/ml colchicine synthesized significantly more IL2R protein, on average, than cells in 80 ng/ml colchicine.

Flow cytometry was used to determine the distribution of IL2R expression among cells in the high-drug and low-drug populations (Figure 5). Cells in each population were reacted with the anti-Tac antibody and then with FITC-conjugated goat anti-mouse antibody. The curves in Figure 5 show number of cells (y-axis) versus fluorescent intensity (x-axis) and thus represent the distribution of the level of cell-surface IL2R expression within the respective populations.

The results of this analysis indicate that even after long-term culturing at 80 ng/ml of colchicine, greater than 80% of the drug-resistant cells also expressed IL2R. Although there was a broad range of the level of IL2R expression, a significant number of these cells expressed very high amounts of the protein on their cell surface. When compared with standards of known IL2R number, the average number of receptors per cell in this population was about 280,000. At 640 ng/ml colchicine, on the other hand, the level of IL2R expression was more uniformly at the high end of the range. This population had an average of 740,000 receptors/cell. Therefore, after selecting cells in increasing concentrations of colchicine, there was an enrichment for cells which were very drug resistant and which profusely expressed IL2R. The net result was a coordinate increase in expression of the MDR1 cDNA and of the non-selected gene (cell-surface receptor).

In short, the results presented herein using the IL-2 receptor as a test gene, clearly demonstrate significant expression of the protein (IL2R) under the control of the SV40 promoter. Expression was efficiently amplified by growing the transfected cells in high concentrations of selective agent.

The MDR1 gene has the advantage of being easily selectable in a variety of cell types. Ueda et al. describe the isolation of a full-length cDNA for the human MDR1 gene and its expression in mouse NIH 3T3 and human KB cell, conferring the selectable multidrug resistance phenotype (Proc. Natl. Acad. Sci. (1987), 84, 3004-3008). In addition, the amplification process proceeds readily and rapidly. Since the MDR1 gene product is a cell surface molecule, it provides a means for selecting transfected cells via fluorescence-activated or magnetic bead cell sorting. Furthermore, the MDR1 system can be used either by cotransfecting the pHaMDR1 plasmid and a non-selectable gene present on a separate plasmid, or by including the desired cDNA within the transcription unit of pSK1.MDR as demonstrated here, or by fusing the non-selected protein to the carboxy terminus of P-glycoprotein (Germann et al, 1989, J. Biol. Chem. 264(13), 7418-7424. Germann et al. disclose the expression of a multidrug resistance-adenosine deaminase fusion gene in human KB cells, yielding a chimeric fusion protein.

Of course, having demonstrated herein the simultaneous overexpression of a non-selectable IL2R cDNA with the selectable MDR1 cDNA, other non-selectable genes can be similarly coexpressed in suitable transfectable cells as will be suggested to one of ordinary skill in the art.

A number of selective agents other than colchicine, such as vinblastine, adriamycin and the like can also be conveniently employed.

## Claims (Claims for the following Contracting State(s): AT, BE, CH, LI, DE, DK, FR, GB, IT, LU, NL, SE)

1. A recombinant plasmid expression vector comprising a human MDR1 gene at a first site and a second separate site for the insertion of a non-selectable gene, wherein when said non-selectable gene is inserted in said second separate site in the expression vector, and a cell is transfected with the plasmid vector containing said non-selectable gene, said non-selectable gene is simultaneously amplified and overexpressed along with the MDR1 gene to obtain two separate and distinct expression products.

2. A plasmid vector as claimed in claim 1 having the characteristics of ATCC deposit number 68011.

3. A plasmid vector as claimed in claims 1 or 2, further comprising a non-selectable gene inserted at said site.

4. A plasmid as claimed in claim 3, wherein the non-selectable gene is an interleukin 2 receptor gene.

5. A cell transfected with a plasmid vector as claimed in any of claims 1-4.

6. A method for identifiable expression of a non-selectable gene, comprising inserting the cDNA of a non-selectable gene desired to be expressed into a plasmid expression vector according to claim 1, transfecting said expression vector into cells and identifying the expression of said non-selectable gene in said cells by the dominant selectable MDR phenotype with which said non-selectable gene is simultaneously overexpressed.

7. A method as claimed in claim 6 for amplified production of a non-selectable gene product, further comprising the step of recovering the product overexpressed by the non-selectable gene by conventional isolation and purification means.

8. A recombinant plasmid expression vector as claimed in claims 1 or 2, for use in constructing a plasmid vector having a non-selectable gene inserted at said second separate site.

## Claims (Claims for the following Contracting State(s): ES)

1. A method for constructing a recombinant plasmid expression vector comprising a human MDR1 gene at a first site and a second separate site for the insertion of a non-selectable gene, wherein when said non-selectable gene is inserted in said second separate site in the expression vector, and a cell is transfected with the plasmid vector containing said non-selectable gene, said non-selectable gene is simultaneously amplified and overexpressed along with the MDR1 gene to obtain two separate and distinct expression products, said method involving the use of a recombinant DNA technique.

2. A method as claimed in claim 1, wherein the plasmid vector has the characteristics of ATCC deposit number 68011.

3. A method as claimed in claims 1 or 2, further comprising the step of inserting a non-selectable gene into said plasmid vector at said second separate site.

4. A method as claimed in claim 3, wherein the non-selectable gene is an interleukin 2 receptor gene.

5. A method for producing a cell transfected with a plasmid vector as claimed in any of claims 1-4, comprising the step of introducing a plasmid vector into a host cell which it is desired to transfect.

6. A method for identifiable expression of a non-selectable gene, comprising the steps of:
inserting the cDNA of a non-selectable gene desired to be expressed into a plasmid expression vector;
transfecting said expression vector into cells; and
identifying the expression of said non-selectable gene in said cells by the dominant selectable MDR phenotype with which said non-selectable gene is simultaneously overexpressed;
wherein the plasmid expression vector comprises a human MDR1 gene at a first site and a second separate site for the insertion of a non-selectable gene and, when said non-selectable gene is inserted in said second separate site and a cell is transfected with the plasmid vector containing said non-selectable gene, said non-selectable gene is simultaneously amplified and overexpressed along with the MDR1 gene to obtain two separate and distinct expression products;

7. A method as claimed in claim 6 for amplified production of a non-selectable gene product, further comprising the step of recovering the product overexpressed by the non-selectable gene by conventional isolation and purification means.

## Patentansprüche (Patentansprüche für folgende(n) Vertragsstaat(en): AT, BE, CH, LI, DE, DK, FR, GB, IT, LU, NL, SE)

1. Rekombinanter Plasmid-Expressionsvektor, der ein menschliches MDR1-Gen an einer ersten Stelle und einer davon getrennten zweiten Stelle für die Insertion eines nicht selektierbaren Gens aufweist, wobei, wenn das nicht selektierbare Gen an der getrennten zweiten Stelle in den Expressionsvektor eingefügt wird und eine Zelle mit dem das nichtselektierbare Gen enthaltenden Plasmidvektor transfektiert wird, das nicht selektierbare Gen mit dem MDR1-Gen gleichzeitig amplifiziert und überexprimiert wird, um zwei gesonderte und verschiedene Expressionsprodukte zu erhalten.

2. Plasmidvektor nach Anspruch 1 mit den Eigenschaften der ATCC-Hinterlegung Nr. 68011.

3. Plasmidvektor nach Anspruch 1 oder 2, der außerdem ein an der genannten Stelle eingefügtes nicht selektierbares Gen aufweist.

4. Plasmidvektor nach Anspruch 3, wobei das nicht selektierbare Gen ein Interleukin-2-Rezeptorgen ist.

5. Mit einem Plasmidvektor nach einem der Ansprüch 1 bis 4 transfektierte Zelle.

6. Verfahren zur identifizierbaren Expression eines nicht selektierbaren Gens, welches das Einfügen der cDNA eines nicht selektierbaren Gens, das exprimiert werden soll, in einen Plasmid-Expressionsvektor nach Anspruch 1, die Transvektion des Expressionsvektors in Zellen, und das Identifizieren der Expression des nicht selektierbaren Gens in diesen Zellen durch den dominanten selektierbaren MDR-Phenotyp umfaßt, mit welchem das nicht selektierbare Gen gleichzeitig überexpremiert wird.

7. Verfahren nach Anspruch 6 zur amplifizierten Produktion eines nicht selektierbaren Genprodukts, das weiter den Schritt der Wiedergewinnung des überexprimierten Produkts durch das nicht selektierbare Gen durch konventionelle Isolierungs- und Reinigungsmaßnahmen umfaßt.

8. Rekombinanter Plasmid-Expressionsvektor nach Anspruch 1 oder 2 zur Verwendung bei der Konstruktion eines Plasmidvektors mit einem an der genannten getrennten zweiten Stelle eingefügten nicht selektierbaren Gen.

## Patentansprüche (Patentansprüche für folgende(n) Vertragsstaat(en): ES)

1. Verfahren zur Konstruktion eines rekombinanten Plasmid-Expressionsvektors, der ein menschliches MDR1-Gen an einer ersten Stelle und an einer gesonderten zweiten Stelle für die Insertion eines nicht selektierbaren Gens aufweist, wobei, wenn das nicht selektierbare Gen an der gesonderten zweiten Stelle im Expressionsvektor eingefügt wird und eine Zelle mit dem das nicht selektierbare Gen enthaltenden Plasmidvektor transfektiert wird, das nicht selektierbare Gen zusammen mit dem MDR1-Gen gleichzeitig amplifiziert und überexpremiert wird, um zwei gesonderte und verschiedene Expressionsprodukte zu erhalten, wobei das Verfahren die Anwendung einer rekombinanten DNA-Technik umfaßt.

2. Verfahren nach Anspruch 1, wobei der Plasmidvektor die Eigenschaften der ATCC-Hinterlegung Nr. 68011 aufweist.

3. Verfahren nach Anspruch 1 oder 2, das außerdem den Schritt des Einfügens eines nicht selektierbaren Gens in den Plasmidvektor an der gesonderten zweiten Stelle umfaßt.

4. Verfahren nach Anspruch 3, wobei das nicht selektierbare Gen ein Interleukin-2-Rezeptorgen ist.

5. Verfahren zum Herstellen einer mit einem Plasmidvektor nach einem der Ansprüche 1 bis 4 transfektierten Zelle, das den Schritt des Einführens eines Plasmidvektors in eine Wirtszelle umfaßt, in welche er transfektiert werden soll.

6. Verfahren zur identifizierbaren Expression eines nicht selektierbaren Gens, mit den Schritten:
Einfügen der cDNA eines nicht selektierbaren Gens, das exprimiert werden soll, in einen Plasmid-Expressionsvektor,
Transfektieren des Expressionsvektors in Zellen, und
Identifizieren der Expression des nicht selektierbaren Gens in den Zellen durch den dominanten selektierbaren NDR-Phenotyp, mit welchem das nicht selektierbare Gen gleichzeitig überexpremiert wird,
wobei der Plasmid-Expressionsvektor ein menschliches MDR1-Gen an einer ersten Stelle und an einer gesonderten zweiten Stelle für das Einfügen eines nicht selektierbaren Gens enthält und, wenn das nicht selektierbare Gen an der gesonderten zweiten Stelle eingefügt ist und eine Zelle mit dem das nicht selektierbaren Gen transfektiert wird, das nicht selektierbare Gen zusammen mit dem NDR1-Gen gleichzeitig amplifiziert und überexprimiert wird, um zwei gesonderte und verschiedene Expressionsprodukte zu erhalten.

7. Verfahren nach Anspruch 6 für die amplifizierte Produktion eines nicht selektierbaren Genprodukts, das außerdem den Schritt der Wiedergewinnung des durch das nicht selektierbare Gen überexprimierten Produkts durch herkömmliche Isolierungs- und Reinigungsmaßnahmen umfaßt.

## Revendications (Revendications pour l'(les) Etat(s) contractant(s) suivant(s): AT, BE, CH, LI, DE, DK, FR, GB, IT, LU, NL, SE)

1. Vecteur d'expression plasmidique recombinant comprenant un gène humain MDR1, à un premier site et à un second site indépendant, pour l'insertion d'un gène ne pouvant pas être sélectionné, dans lequel, quand ledit gène ne pouvant pas être sélectionné est inséré dans ledit second site indépendant du vecteur d'expression, et quand une cellule est transfectée par le vecteur plasmidique contenant ledit gène ne pouvant pas être sélectionné, ce dernier est simultanément amplifié et surexprimé avec le gène MDR1, pour obtenir deux produits d'expression distincts et indépendants.

2. Vecteur plasmidique selon la revendication 1 présentant les caractéristiques du dépôt effectué auprès de l'ATCC sous le numéro 68011.

3. Vecteur plasmidique selon la revendication 1 ou 2, comprenant en outre un gène ne pouvant pas être sélectionné, inséré audit site.

4. Plasmide selon la revendication 3, dans lequel le gène ne pouvant pas être sélectionné est le gène du récepteur de l'interleukine-2.

5. Cellule transfectée par un vecteur plasmidique selon l'une quelconque des revendications 1 à 4.

6. Procédé pour exprimer, de manière identifiable, un gène ne pouvant pas être sélectionné, comprenant l'insertion de l'ADNc d'un gène ne pouvant pas être sélectionné, destiné à être exprimé dans un vecteur d'expression plasmidique selon la revendication 1, la transfection des cellules par ledit vecteur d'expression et l'identification de l'expression dudit gène ne pouvant pas être sélectionné dans lesdites cellules, par le phénotype dominant pouvant être sélectionné MDR avec lequel ledit gène ne pouvant pas être sélectionné est simultanément surexprimé.

7. Procédé selon la revendication 6 pour amplifier la production du produit d'un gène ne pouvant pas être sélectionné, comprenant en outre l'étape de récupération du produit surexprimé par le gène ne pouvant pas être sélectionné, à l'aide de moyens d'isolement et de purification conventionnels.

8. Vecteur d'expression plasmidique recombinant selon les revendications 1 ou 2, destiné à être utilisé pour construire un vecteur plasmidique possédant un gène ne pouvant pas être sélectionné, inséré audit second site indépendant.

## Revendications (Revendications pour l'(les) Etat(s) contractant(s) suivant(s): ES)

1. Procédé pour construire un vecteur d'expression plasmidique recombinant comprenant un gène humain MDR1, à un premier site et à un second site indépendant, pour l'insertion d'un gène ne pouvant pas être sélectionné, selon lequel, quand ledit gène ne pouvant pas être sélectionné est inséré dans ledit second site indépendant, du vecteur d'expression, et quand une cellule est transfectée par le vecteur plasmidique contenant ledit gène ne pouvant pas être sélectionné, ce dernier est simultanément amplifié et surexprimé avec le gène MDR1, pour obtenir deux produits d'expression distincts et indépendants, ledit procédé mettant en oeuvre une technique d'ADN recombinant.

2. Procédé selon la revendication 1 selon lequel le vecteur plasmidique présente les caractéristiques du dépôt effectué auprès de l'ATCC sous le numéro 68011.

3. Procédé selon la revendication 1 ou 2 comprenant en outre l'étape d'insertion d'un gène ne pouvant pas être sélectionné, dans ledit vecteur plasmidique, audit second site indépendant.

4. Procédé selon la revendication 3, selon lequel le gène ne pouvant pas être sélectionné est un gène du récepteur de l'interleukine-2.

5. Procédé pour produire une cellule transfectée par un vecteur plasmidique selon l'une quelconque des revendications 1 à 4, comprenant l'étape d'introduction d'un vecteur plasmidique dans une cellule hôte destinée à être transfectée.

6. Procédé pour exprimer de manière identifiable un gène ne pouvant pas être sélectionné, comprenant les étapes suivantes :
insertion de l'ADNc d'un gène ne pouvant pas être sélectionné, destiné à être exprimé dans un vecteur d'expression plasmidique;
transfection de cellules par ledit vecteur d'expression ; et
identification de l'expression dudit gène ne pouvant pas être sélectionné dans lesdites cellules, par le phénotype dominant MDR pouvant être sélectionné, avec lequel ledit gène ne pouvant pas être sélectionné est simultanément surexprimé;
procédé selon lequel le vecteur d'expression plasmidique comprend un gène humain MDR1, à un premier site et à un second site indépendant, pour l'insertion d'un gène ne pouvant pas être sélectionné et, quand ledit gène ne pouvant pas être sélectionné est inséré dans ledit second site indépendant, et quand une cellule est transfectée par le vecteur plasmidique contenant ledit gène ne pouvant pas être sélectionné, ce dernier est simultanément amplifié et surexprimé avec le gène MDR1 pour obtenir deux produits d'expression distincts et indépendants.

7. Procédé selon la revendication 6 pour amplifier la production du produit d'un gène ne pouvant pas être sélectionné, comprenant en outre l'étape de récupération du produit surexprimé par le gène ne pouvant pas être sélectionné, en mettant en oeuvre des moyens d'isolement et de purification conventionnels.
